(19)

Europäisches
Patentamt
European
Patent Office
Office européen
des brevets

(11) **EP 4 242 658 A1**

(12) **EUROPEAN PATENT APPLICATION**

(43) Date of publication:
 **13.09.2023  Bulletin 2023/37**

(21) Application number: **22161335.9**

(22) Date of filing: **10.03.2022**

(51) International Patent Classification (IPC):
 **G01N 33/543** (2006.01)   **G01N 33/50** (2006.01)

(52) Cooperative Patent Classification (CPC):
 **G01N 33/5005; G01N 33/50; G01N 33/5044**

(84) Designated Contracting States:
 **AL AT BE BG CH CY CZ DE DK EE ES FI FR GB GR HR HU IE IS IT LI LT LU LV MC MK MT NL NO PL PT RO RS SE SI SK SM TR**
 Designated Extension States:
 **BA ME**
 Designated Validation States:
 **KH MA MD TN**

(71) Applicant: **Max-Planck-Gesellschaft zur Förderung der Wissenschaften e.V.**
 **80539 München (DE)**

(72) Inventors:
 • **KRÄTER, Martin**
  **91054 Erlangen (DE)**
 • **GUCK, Jochen Reinhold**
  **91052 Erlangen (DE)**

 • **HOFEMEIER ABU HATTUM, Shada**
  **91052 Erlangen (DE)**
 • **KUBÁNKOVÁ, Markéta Icha**
  **91054 Erlangen (DE)**
 • **HOHBERGER, Bettina**
  **91245 Simmelsdorf (DE)**
 • **MARDIN, Christian**
  **91056 Erlangen (DE)**
 • **WALLUKAT, Gerd Georg Wilhelm**
  **13129 Berlin (DE)**

(74) Representative: **Hoffmann Eitle Patent- und Rechtsanwälte PartmbB Arabellastraße 30 81925 München (DE)**

Remarks:
 Amended claims in accordance with Rule 137(2) EPC.

(54) **A SYSTEM AND METHOD FOR BIOMOLECULE DETECTION**

(57)   The present invention relates to a method of detecting biomolecules in a sample, the method comprising:
- adding cells to the sample, the cells being arranged so that the biomolecules to be detected can interact with the cells to thus change one or more properties of the cells,
- detecting the properties of the cells, and
- comparing the detected properties with reference values to infer the presence or absence and/or concentration of the biomolecules.

Figure 1/4

EP 4 242 658 A1

## Description

## Technical field

The present invention relates to a system and method for detecting biomolecules in a sample.

## Technical background

In the field of biological, biotechnological, medical and diagnostic procedures, the characterization of a sample by detecting biomolecules is an important starting point for numerous applications. This includes monitoring the presence or absence of biomolecules and changes thereof in samples for biochemical research, medical diagnosis, predicting therapeutic responses, understanding disease pathogenesis, and drug development. For example, when a new pathogen appears, analysing samples from infected individuals by detecting biomolecules can aid in the diagnosis of the associated disease, in the prevention or containment of outbreaks, in the process of monitoring disease progression or post-disease symptoms and in the development of appropriate therapeutic intervention.

The limitations of available approaches for biomolecule detection were highlighted during the coronavirus disease-19 (COVID-19) pandemic which is an ongoing global pandemic. COVID-19 is caused by severe acute respiratory syndrome coronavirus 2 (SARS-CoV-2) which affects the pulmonary systems and impairs microcirculation (acute phase). Four or more weeks after first being infected with SARS-CoV-2 (see, e.g., Post-COVID Conditions | CDC; NICE , www.nice.org.uk/guidance/ng188), some patients experience persisting post-COVID-19 health problems (Long-COVID-19, also termed Post-Acute Sequelae of SARS-CoV-2 infection (PASC)) such as sequelae in the respiratory system, nervous system and neurocognitive disorders, mental health disorders, metabolic disorders, cardiovascular disorders, gastrointestinal disorders, malaise, fatigue, musculoskeletal pain and anaemia (as described in Al-Aly et al., doi: 10.1038/s41586-021-03553-9). The underlying mechanism may be driven by an aberrant immune response, hyperactivation of the immune system or autoimmunity. Specifically, patients with the SARS-CoV-2 infection have higher levels of autoantibodies than uninfected individuals. The autoantibodies impair the patient's immune system (e.g., by attacking B cells, eliminating interferons (IFNs)) or attack the patient's own tissues (e.g., by targeting proteins in their blood vessels, connective tissue and brain).

Also, other diseases are characterized by the immune system attacking the patient's own tissues (autoimmunity) including type 1 diabetes (T1D), systemic lupus erythematosus (SLE), multiple sclerosis (MS), rheumatoid arthritis (RA), Graves' disease, Hashimoto's thyroiditis, Alzheimer's disease, Huntington's disease, inflammatory bowel disease (IBD), Crohn's disease, and coeliac disease (as described in, e.g., [6] Burbelo and O'Hanlon, doi: 10.1097/BOR.0000000000000107; [8] Morgenthaler et al., doi: 10.1055/s-2007-978858). A further relevant publication in that respect is Wallukat et al., Anti-β1-Adrenoceptor Autoantibodies with Chronotropic Activity from the Serum of Patients with Dilated Cardiomyopathy: Mapping of Epitopes in the First and Second Extracellular Loops, J. Mol. Cell Cardiol. 27, 397-406.

The presence of biomolecules in a sample (such as autoantibodies in a blood sample) may be detected using specific antibody-antigen interactions. In an enzyme-linked immunosorbent assay (ELISA), the binding to the target protein or peptide is investigated. Despite its wide application, ELISA has several limitations including an insufficient level of sensitivity (e.g., due to the use of a single antibody) and a low signal to background noise level (e.g., due to cross-reactivity if two antibodies are used, or non-specific binding of antibodies to plastic surfaces).

Higher sensitivity may be achieved in complex multi-step screening strategies wherein ELISA is combined with immunofluorescence and other assays. For example, to detect β1-adrenergic receptor autoantibodies in patients with cardiomyopathy (a disease of the heart muscle), ELISA was combined with immune-fluorescence and cyclic adenosine monophosphate (cAMP) responses in cells expressing native human β-adrenergic receptors (β-ARs) which may be detected by exchange protein activated by cAMP (Epac) fluorescence resonance energy transfer (FRET) (Nikolaev et al., doi: 10.1016/j.jacc.2007.03.051) or by assays to determine protein kinase A (PKA) activity (SignaTECT PKA assay) (Yu, Stavrakis et al., doi: 10.1016/j.jash.2011.10.003). Despite the improvement of sensitivity over ELISA, these complex multi-step screening strategies are limited to specific applications and do not allow to simultaneously detect the presence of different types of biomolecules in a sample.

Available approaches for biomolecule detection are currently suffering from various limitations including low sensitivity, high costs and long procedure durations (as described in [1], Drucker and Krapfenbauer, doi: 10.1186/1878-5085-4-7; [2], Mayeux, doi: 10.1602/neurorx.1.2.182; [3], Nimse et al., doi: 10.1039/c5an01790d).

Accordingly, there is a need in the art for a new system and methods for detection of the presence, absence or concentration of biomolecules such as autoantibodies in a sample. Furthermore, a new system and methods for efficient real-time detection of biomolecules are needed to properly diagnose and treat diseases or post-disease symptoms.

## Summary

The present invention meets or addresses at least some of the above needs and aims at solving the above problems in the art by providing systems and methods which are defined by the independent claims. Preferred embodiments are set out in the respective dependent claims, as well as in the embodiments described below.

The present invention includes a method of detecting one or more biomolecules in a sample. The method includes the steps of: (i) combining the sample with cells (e.g., by adding cells to the sample), (ii) detecting at least one property of the cells which changes in response to the biomolecule, and (iii) inferring the presence, absence and/or concentration of the biomolecules. Changes are determined relative to a control, e.g., a reference value for the property of the cells.

One aspect of the present invention is the detection of biomolecules which are associated with a specific condition in a sample obtained from a patient (e.g., suspected of suffering from a disease, post-disease symptoms, recovering from a disease, immunisation by vaccination, treatment, etc.). To detect the presence of a biomolecule, cells are arranged so that the biomolecule to be detected can interact with the cells to thus change one or more properties of the cells. The interaction of the biomolecule and the cells may feature binding of the biomolecule to a receptor which is involved in pathogenesis of the disease. The present system and method enable clinical phenotyping of a patient, provide high diagnostic accuracy, and may yield insights into disease pathogenesis or suitable treatment options.

One aspect of the present invention is the detection of autoantibodies in a body fluid of a patient (e.g., blood, saliva, urine, or cerebrospinal fluid). Autoantibodies which are produced under physiological or pathophysiological conditions may act as an indicator of a disease-promoting condition, a disease such as an autoimmune disease, or post-disease symptoms such as long term neurological and/or cardiological symptoms. Upon addition of a sample with autoantibodies to cells (e.g., mammalian cells) which express receptors for the autoantibodies (e.g., cell surface molecules such as proteins with extracellular regions), the autoantibodies and the receptors interact and thus change one or more properties of the cells. By comparing the detected properties with a reference value, the presence or absence of the autoantibodies is inferred. The present system and methods detect autoantibodies with high specificity, sensitivity, reproducibility and sample throughput in a sample.

One aspect of the present invention is detecting a change of at least one property of the cells in the presence or absence of biomolecules by using a microfluidic channel. The microfluidic channel may be part of a microfluidic device (e.g., a chip) through which a sample and cells can be analysed by continuous phenotyping of cells in a contactless manner in less than 0.001 seconds.

One aspect of the present invention includes a method of detecting at least two different types of biomolecules (e.g. antibodies) simultaneously in a sample. This includes adding a sample comprising at least two different biomolecules to a cell population (e.g. mammalian cells) comprising at least two cell subpopulations or cells having a different origin. A cell subpopulation/group of cells having a different origin may be genetically modified for detecting a biomolecule, wherein the cell comprises (i) a heterologous nucleic acid encoding a fluorescent molecule for identifying the cell, wherein, in the presence of the biomolecule, the behaviour of the fluorescent molecule is not affected; and (ii) a natural or heterologous nucleic acid encoding a receptor for specific binding of the biomolecule, wherein, in the presence of the biomolecule, the receptor changes at least one property of the cell which can be detected (e.g., cell mass density, cell size, or mechanical properties). In the cell population, the at least two modified cells differ in features (i) and (ii). Both features may be detected simultaneously, e.g., by simultaneous mechanical characterization and fluorescence characterization. The cells of different origin can only bind to one specific biomolecule and have originally a different size or are labelled with a different fluorescence marker.

Thus, the present invention provides systems and methods for biomolecule detection by providing the preferred embodiments described below:

1. A method of detecting biomolecules in a sample, the method comprising:

- adding cells to the sample, the cells being arranged so that the biomolecules to be detected can interact with the cells to thus change one or more properties of the cells,

- detecting the properties of the cells, and

- comparing the detected properties with reference values to infer the presence or absence and/or concentration of the biomolecules.

2. The method according to embodiment 1, wherein the detection of the properties of the cells takes place in a microfluidic channel, with the sample being led through the microfluidic channel, wherein preferably, the detected properties comprise mechanical properties, and wherein the cells are preferably deformed inside the channel.

3. The method according to embodiment 1 or 2, wherein the cells are arranged so that the interaction of the biomolecules with the cells is binding of the biomolecules to a receptor of the cells.

4. The method according to one of the preceding embodiments, wherein the cells are arranged so that more than one type of biomolecules can interact with the cells and wherein different types of biomolecules lead to different detectable changes of properties of the cells.

5. The method according to one of the preceding embodiments, wherein the properties include one or more of the size, shape, structure, morphology, passage time, mass density, elastic and inelastic light scattering properties, such as Raman and/or Brillouin scattering, dielectric and acoustic impedance and/or deformability, elasticity and viscosity of the cells.

6. The method according to one of the preceding embodiments, wherein the cells are mammalian cells, and
optionally wherein the cells comprise or consist of natural cells, transfected cells, and/or synthetic cells.

7. The method according to one of the preceding embodiments, wherein the cells are configured to change their properties when exposed to the biomolecules, and wherein the biomolecules are antibodies interacting with a receptor of the cells, preferably a cell surface molecule, optionally wherein the antibodies are autoantibodies that target G protein coupled receptors (GPCRs).

8. The method according to one of the preceding embodiments, further comprising using the detected properties to infer the concentration of biomolecules in the sample.

9. The method according to one of the preceding embodiments, wherein the sample is a fluid, preferably a liquid;

optionally wherein the fluid is obtained from a subject, further optionally wherein the fluid is saliva, blood, excreta, and/or a body fluid, preferably wherein the excreta is urine, and/or wherein the body fluid is cerebrospinal fluid (CSF); and

optionally wherein the subject is a mammal.

10. A method of detecting at least two types of biomolecules in a sample, the method comprising:

- adding a cell population to the sample; wherein the cell population comprises at least two cell subpopulations or at least two groups of cells from different originas:

the first cell subpopulation/group is arranged so that the first type of biomolecules to be detected can interact with cells of the first cell subpopulation/group, and the first cell subpopulation/group is characterized by a fluorescence behaviour,

and the second cell subpopulation/group is arranged so that the second type of biomolecules to be detected can interact with cells of the second cell subpopulation/group and the second cell subpopulation/group is characterized by a different fluorescence behaviour as compared to the first cell subpopulation/group; and

wherein the cells being arranged so that the biomolecules to be detected can interact with the cells to thus change one or more properties of the cells;

- detecting the properties of the cells and the fluorescence behaviour of the cells;

- comparing the detected properties with reference values to infer the presence or absence of the biomolecules;

- comparing the fluorescence behaviour of the cells to assign the change of the detected properties to the first or the second type of cells; and

- to infer the presence or absence of the first type of biomolecules and the presence or absence of the second type of biomolecules.

11. The method according to embodiment 10, wherein the types of biomolecules are biomolecules which interact differently with the cells,
optionally wherein the biomolecules bind different receptors of the cell.

12. The method according to embodiments 10 or 11, wherein the biomolecules are antibodies and the types of biomolecules are antigenically distinct antibodies, wherein the first type of antibody can interact with cells of the first cell subpopulation/group but not with cells of the second cell subpopulation/group, and the second type of antibody can interact with cells of the second cell subpopulation/group but not with cells the first cell subpopulation/group.

13. The method according to one of embodiments 10 to 12, wherein the detected properties of the cells are mechanical properties, and wherein the mechanical properties and the fluorescence behaviour of the cells are detected in a microfluidic channel.

14. A kit arranged for detecting biomolecules, comprising:

- one or more cells, the cells being arranged so that the biomolecules to be detected can interact with the cells to thus change one or more properties of the cells,
- a means for detecting the one or more properties of the cells, the kit preferably being arranged for being used in the method of one of the preceding claims.

15. The kit for use in a method of detecting one or more biomolecules in a sample obtained from a subject of embodiment 14, preferably wherein the sample is saliva, blood, urine, or cerebrospinal fluid (CSF).

**Description of the drawings**

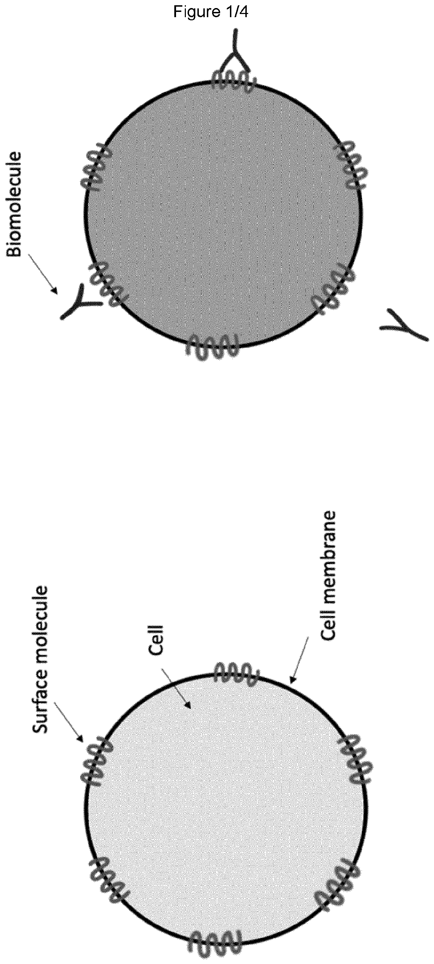

Figure 1 is a set of schematic drawings showing the detection of a biomolecule, exemplified by an antibody. Upon interaction of the biomolecule with a receptor of the cell (e.g. a cell surface molecule), a change in one or more properties of the cells can be detected.

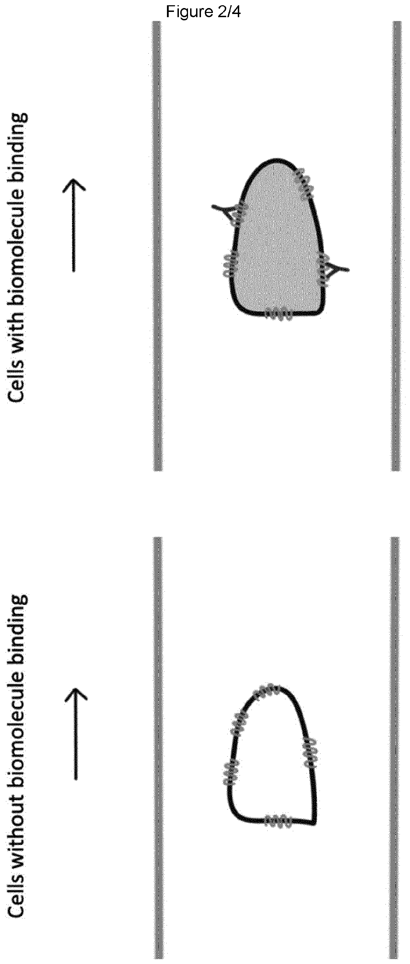

Figure 2 illustrates the system of passing cells expressing surface molecules through a microfluidic channel in the presence or absence of the biomolecules to be detected.

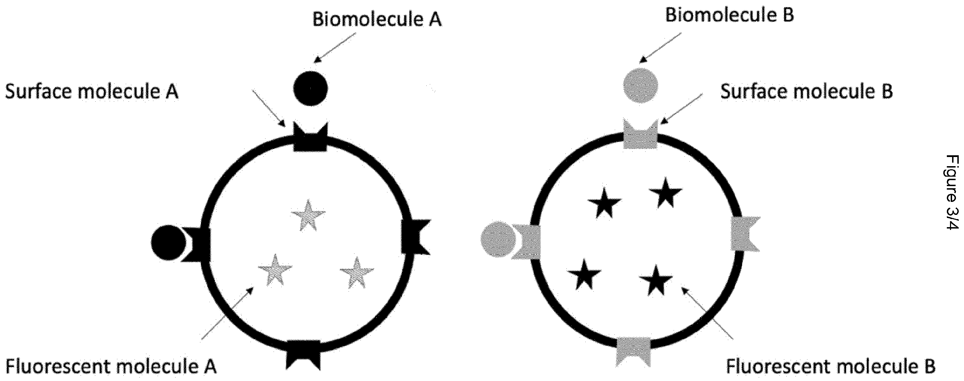

Figure 3 exemplifies the detection of at least two different types of biomolecules in a sample by a cell population comprising at least two cell subpopulations. To detect, e.g., two different biomolecules A and B in a sample, two cell subpopulations A and B may be used. Cell subpopulation A expresses (i) receptor A specific for biomolecule A and (ii) a fluorescent molecule A specific for cell A. Cell subpopulation B expresses (i) receptor B specific for biomolecule B and (ii) a fluorescent molecule B specific for cell B. When combining the read-out of the changes in the physical properties of the cells (e.g., cell mass density, cell size, or mechanical properties) and the fluorescent signals of cell subpopulations A and B, the presence of biomolecules A and/or B in a pool of cells may be inferred.

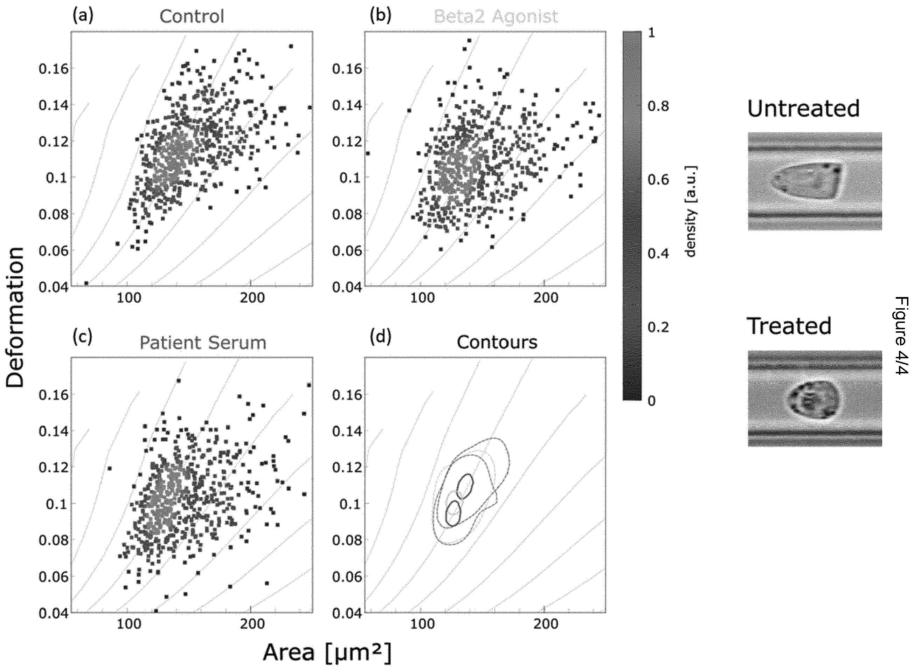

Figure 4 exemplifies the present disclosure by using real-time deformability cytometry (RT-DC) for cell mechanical characterization of human cells upon incubation with a biomolecule. The biomolecule is an agonist for a G-protein coupled receptor (GPCR), i.e., a beta2 adrenoreceptor. Specifically, HL60 human myeloid leukemia cells were incubated with a drug activating GPCRs, or patient-derived blood serum containing autoantibodies which target GPCRs including beta2 adrenoreceptors. In (a), (b) and (c), deformability cytometry plots are shown which represent the deformation for each cell event with its corresponding measured cell area ($\mu m^2$). **Figure 4(a):** control measurement of HL60 cells without biomolecules addition (reference value). **Figure 4(b):** measurement of HL60 cells incubated with a drug (beta2 agonist). **Figure 4(c):** measurement of HL60 cells incubated with patient blood serum containing beta2 autoantibody. **Figure 4(d):** contour plots of all the measurements in (a), (b) and (c). Images on the right show two representative cell images with (treated) and without (untreated) the addition of the beta2 GPCR specific molecule.

**Detailed description**

*Definitions*

All publications, patents and patent applications cited herein are hereby incorporated by reference in their entirety for all purposes.

In accordance with the present invention, each occurrence of the term *"comprising"* may optionally be substituted with the term *"consisting of"*. The articles "a / an" and *"the"* are used herein to refer to one or to more than one (e.g., to at least one) of the grammatical object of the article unless otherwise clearly indicated by contrast. By way of example, *"an element'* means one element or more than one element. The term "or" is used herein to mean, and is used interchangeably with, the term *"and/or,"* unless context clearly indicates otherwise.

The term *"about'* as used herein refers to a deviation of $\pm$ 10 % from the recited value. When the word *"about'* is used herein in reference to a number, it should be understood that still another embodiment includes that number not modified by the presence of the word *"about'*. In the absence of the term *"about'* and unless the context dictates otherwise, generally accepted rounding rules apply to the specified values.

The term *"biomolecule"* as used herein refers to a substance present in living cells or organisms (e.g., intracellular and extracellular) in physiological or disease state or after a treatment. A biomolecule may be a nucleic acid (an oligonucleotide comprising nucleotides such as deoxyribonucleotides or ribonucleotides), a peptide and protein (such as an antibody/autoantibody), carbohydrates and lipids. In one aspect, a biomolecule may be derived from a sample of a healthy subject to analyse the physiological functions related to natural organ function, homeostasis, aging, or regeneration. Said functions may also be analysed upon changes in the environment. In other instances, a biomolecule may be derived from an area of interest after injury to analyse temporal or permanent changes upon wounding, fibrosis or scar formation. In other instances, biomolecules may also be derived from vaccinated individuals and compared with samples of non-vaccinated or infected individuals. In other instances, biomolecules may also be derived from pathological situations like abnormal organ development, autoimmune diseases, chronic diseases, infectious disease or cancer and compared with samples of healthy or cured individuals. Furthermore, biomolecules may be clinical markers of a disease or disorder (e.g., immune biomarker abnormalities, clinical severity markers), predictive markers of a disease (e.g., markers of thrombo-inflammatory activation) or therapeutic biomarkers. A biomolecule may be a ligand (agonist or antagonist) specific for a natural or synthetic receptor of a cell.

The term "*at least two types of biomolecules*" as used herein refers to biomolecules which interact differently with the cells, e.g. by binding to different receptors of the cells. The different interaction of the types of biomolecules may lead to detectable changes of the same or different properties of the cells when comparing to a reference value (e.g., for both types of biomolecules, morphological and rheological parameters are quantified such as cell size, elasticity or deformability). In some instances, at least two types of biomolecules may be characterized in that they have a different structure. In some instances, at least two types of biomolecules may be antigenically distinct antibodies, e.g. differing in their amino acid sequence of one or more CDRs of each of the variable domains of the light and heavy chain, target antigen, functional features and/or the set of specific amino acids of the antigen which are specifically recognised and bound by the antibody (epitope).

The term "*sample*" as used herein refers to a composition comprising at least one biomolecule of interest. A sample may be processed prior to its analysis. For example, a sample may be processed (e.g., lyophilized or frozen) for transportation and/or storage. A sample may be a diagnostic sample for identifying a patient group which is characterized, e.g., by an infection or a disease. A sample may comprise, one, two or more biomolecules of interest such as nucleic acids, proteins, carbohydrates, lipids and combinations thereof.

The term "*fluid*" as used herein refers to a solution, e.g., a liquid sample. A fluid is characterized by its composition, e.g., comprising at least one or more biomolecules, its chemical composition (e.g., ion concentration), osmolarity (number of solute particles per 1 L of solvent, temperature dependent), pH as well as its ability to effect a cell suspended in the fluid (e.g., tonicity which takes into consideration the ability of a fluid to cross a cell membrane; osmotic pressure etc.). The fluid may be a body fluid obtained from a subject such as saliva, blood (e.g., serum, plasma, cerebrospinal fluid), and/or excreta (e.g., urine, faeces) for detecting a biomolecule of interest. A patient derived body fluid may be used to aid in the diagnosis or therapy of a disease. Furthermore, it may be used for detecting or monitoring of a disease (e.g., autoimmune disease, infectious disease), assessing risks and/or efficiency of a treatment, prevention or genetic screening. Body fluids may comprise hormones, steroids, antibodies, growth factors, cytokines and chemokines, nucleic acids, proteins and/or drugs.

The term "*cell*" as used herein refers to an individual cell of any cell type. Specifically, a cell is characterized in that it can change one or more properties of the cells (such as morphological and rheological parameters for characterizing biomechanical features of cells) upon interaction with a biomolecule of interest. A cell may be a eukaryotic cell, preferably a mammalian cell, more preferably a human, mouse, rat, or hamster cell. The cell may be derived from a multicellular organism or parts thereof (e.g. tissue of an animal). A cell may be characterized by its age, developmental stage and physiological or disease state. A cells used for detection of a biomolecule may naturally express surface molecules that interact specifically with said biomolecule ("*natural cell*"), for example, endothelial cells, blood-derived neutrophils and monocytes, smooth muscle cells, liver-derived hepatocytes, kidney-derived renal cells and podocytes. Alternatively, the cells can be genetically modified to express natural or heterologous receptors that interact with a biomolecule of interest ("*modified cell*"). For microfluidic-based approaches, a cell has preferably a smooth contour.

The term "*transfected cell*" as used herein refers to a cell wherein deliberately nucleic acids (DNA or RNA), proteins or ribonucleoprotein (RNP) complexes were introduced to modify the cell. Transfection may utilize standard techniques which are known to the skilled person. Transfection may either be stable or transient (for a limited period of time). For stable transfections, foreign nucleic acids may be integrated into the cell's genome.

The term "*synthetic cell*" as used herein refers to a modified cell which is not of natural origin. Synthetic cells are engineered particles that mimic one or more functions of biological cells. Synthetic cells are designed and built to include the surface molecules as well as the specific biochemical machinery that interprets the interaction of the biomolecule with the surface molecule through one or more changes in the physical parameters of the synthetic cells.

The term "*cell population*" as used herein refers to a mixture of at least two cell subpopulations. Each cell subpopulation is characterized by a unique fluorescence behaviour (i.e., it can be distinguished from the fluorescence behaviour of other subpopulations) and a unique interaction with a biomolecule of interest (i.e. a specific type of biomolecules can only interact with one cell subpopulation but not with the others) as compared to the other subpopulations within the cell population. The fluorescence behaviour of each cell subpopulation remains unchanged in the presence or absence of the biomolecule of interest (i.e. the parameter does not vary between sample and reference value). Hence, determining the fluorescence behaviour allows to maintain the identity of the cell subpopulation when determining the properties of the cell. The readout of the unique interaction with a biomolecule of interest is performed by monitoring a change of a property of the cell (such as a mechanical property) in the presence of the biomolecule (i.e. the property varies between sample and reference value).

The term "*subject*" as used herein refers to a multicellular organism such as a mammal. A subject may be a laboratory animal such as a bat, a rodent (e.g., mice, rats, guinea pigs, hamsters etc.), a non-human primate (e.g., macaques, marmosets, tamarins, monkeys, baboons etc.), or a human being. A subject may be characterized by its age, developmental stage and physiological or disease state. For example, the subject may be a patient suspected of suffering from a disease, post-disease symptoms, recovering from a disease, immunisation by vaccination, treatment, etc.

The term "*property of a cell*" as used herein refers to structural and/or functional features of a cell. Structural features may include one or more of cell size, shape, structure, morphology, passage time, mass density, elastic and inelastic light scattering properties, such as Raman and/or Brillouin scattering, dielectric and acoustic impedance and/or deformability and viscosity.

The term "*mechanical property of a cell*" as used herein refers to a cell mechanical phenotype which can be measured, e.g., by traditional methods (e.g., atomic force microscopy, micropipette aspiration, optical stretching, parallel-plate rheology), or microfluidic-based approaches. Microfluidic-based approaches may include constriction-based deformability cytometry (cDC), shear flow deformability cytometry (sDC) such as real-time deformability cytometry (RT-DC), real-time 1D-imaging fluorescence and deformability cytometry (RT-FDC), real-time analysis of physical phenotype in deformational flow (RAPID), and extensional flow deformability cytometry (xDC) (described, e.g., in [5], Urbanska et al., doi: 10.1038/s41592-020-0818-8). Measurements of cell mechanics may circumvent the need for extrinsic labels (e.g., fluorescent dyes). For example, RT-DC is a label-free, high-throughput technology that allows quick image-based mechanical interrogation of cells at rates of several hundred cells per second (see Otto et al., doi: 10.1038/nmeth.3281).

The term "*elasticity*" or "*stiffness*" as used herein refers to a biophysical marker or a mechanical phenotype of a cell. Cellular elasticity indicates the ability of the cell to deform in response to external stress.

The term "*deformability*" as used herein refers to the ability of a cell to change its shape. Deformability is a biophysical marker or a mechanical phenotype of a cell and may be quantified by the parameter cell deformation, d, e.g., measured by deformability cytometry (DC). To this end, a cell may be illuminated with a pulsed, high-power LED (short enough to prevent motion blurring of the cells) and imaged with a complementary metal-oxide semiconductor (CMOS) camera at 2,000-4,000 f.p.s. In constriction-based DC (cDC), cell deformability is defined as the inverse of cell passage time. In extensional flow DC (xDC), cells are stretched by an extensional flow usually associated with a cross slot microfluidic architecture, and cell deformability is defined as the maximal aspect ratio observed in the extensional flow region. In shear flow DC (sDC) such as RT-DC, cells are driven into a funnel-like constriction in a microfluidic channel where they are deformed by shear forces and pressure gradients into a bullet-like shape, and cell deformation is defined as "1 - circularity" (see, e.g., Fig. 1 of [5], Urbanska et al., doi: 10.1038/s41592-020-0818-8).

The term "*passage time*" as used herein refers to a characteristic property of a cell measured by microfluidic-based approaches. For example, in the specific setup of cDC mentioned in Urbanska et al., the passage time of untreated HL60 cells is 23 ms, entailing a throughput of a few cells per second (e.g., [5], Urbanska et al., doi: 10.1038/s41592-020-0818-8).

The term "*physical phenotyping*" as used herein refers to a biophysical marker of a cell including its state and function. The biophysical marker may have a value in clinical diagnostics. The physical phenotyping of cells has been utilized extensively for understanding biological processes taking place in organisms' development or disease. Methods to evaluate and analyze different aspects of the physical phenotyping are known to the skilled person (e.g., [4], Wu et al., doi: 10.1038/s41592-018-0015-1; [5], Urbanska et al., doi: 10.1038/s41592-020-0818-8).

The term "*concentration*" as used herein refers to the ratio between the amount of solute and the amount of solution and describes how many moles exist per unit volume of the final solution (mol/L).

The term "*absense*" as used herein refers to a quantity or concentration of biomolecules which is below the limit of detection that can be detected with the selected means for detecting the property which is changed in the presence of the biomolecules. Methods to evaluate the limit of detection are known to the skilled person.

The term "*receptor*" as used herein refers to a specific interaction or binding site for a biomolecule which upon interaction changes one of more properties of a cell, e.g., by controlling, regulating or adjusting a cellular process. For example, binding of a biomolecule (ligand) to a receptor (e.g., a cell surface molecule) may result in a conformational change (e.g., by modifying its tertiary structure) which may trigger a cascade of events that may leads to a biological response, e.g., activate downstream effectors causing activation or inhibition of specific cellular processes (e.g., signalling pathways). A receptor may be a cell surface molecule, e.g., a protein with extracellular regions, acting as a receptor for a specific biomolecule. Examples of cell surface receptors are G-protein coupled receptors (GPCRs) which detect biomolecules outside a cell (agonists) and activate cellular responses.

The term "*interaction*" as used herein refers to attractive molecular forces between a biomolecule and a cell, e.g., by binding. Interaction may be direct or indirect and results in a change of one or more properties of the cell (e.g., cell such as size, shape, structure, morphology, passage time, mass density, elastic and inelastic light scattering properties, dielectric and acoustic impedance and/or deformability and viscosity) which can be detected (e.g., while a cell passes through a microfluidic channel). Interaction may be observed between a ligand and a receptor, an antibody and an antigen, a biomolecule and a receptor, e.g., a plasma membrane associated molecule such as proteins, sugar residues of glycoproteins or glycolipids, carbohydrate receptors on the cell surface. The interaction of a biomolecule (in a liquid sample) and a cell may depend on chemical composition (e.g., ion concentration), osmolarity (number of solute particles per 1 L of solvent, temperature dependent) and osmolality (number of solute particles in 1 kg of solvent, temperature independent), pH, temperature, pressure, flow rate, conductivity, voltage, and/or current.

The term "*binding*" as used herein refers to an interaction of a biomolecule and a cell. When biomolecules are in the proximity of living cells, an interaction between this molecule and the cells can occur. One form of interaction, but not limited to this one, is the specific binding of a biomolecule such as an antibody to a molecule expressed on the surface of said cell (a receptor). The biomolecule interaction with the living cells may be accompanied with changes in one or more physical properties of the cells. Binding may be based on an electrostatic interaction of just a few residues (e.g., amino acids or sugar residues) and may be sensitive to structural polymorphism or mutation present in the biomolecule. Binding of a biomolecule may result in reversible or irreversible changes of the properties of a cell.

The term "*reference value*" as used herein refers to a value or a set of values used to interpret a value for a test sample (e.g., for a property of a cell such as size, shape, structure, morphology, passage time, mass density, elastic and inelastic light scattering properties, dielectric and acoustic impedance and/or deformability and viscosity). For example, if a sample is tested for the presence of a specific biomolecule, the reference value refers to the value of at least one property measured for a cell under identical conditions (e.g., experimental conditions and settings) in the absence of the specific biomolecule.

The term "*change*" as used herein refers to any form of physical or chemical change. For example, this may include activation or inhibition of a receptor, the effect of a mutation on a receptor (e.g., for probing mutant cells with specific physiological characteristics), the effect of concentration of a receptor and/or of biomolecules.

The term "*detectable changes*" as used herein refers to a difference for a measurable property of a cell which may be brought about as a result of the interaction of a biomolecule and the cell. For example, binding of an agonist (a biomolecule such as an autoantibody) to a G-protein coupled receptor (GPCR) causes a conformational change in the GPCR and downstream signal transduction which may be detected by, e.g., RT-DC analysis, e.g., as described in Urbanska et al., doi: 10.1016/bs.mcb.2018.06.009. Such a detectable change is strong enough that it can be observed externally from studying individual cells or cell populations.

The term "*means for detecting*" as used herein refers to an analytic system for physical phenotyping of a cell. Detection may be performed by frequency shift and/or imaging combined with real-time or subsequent analysis of the detected phenotype. Phenotyping may be performed in less than 0.001 seconds. For microfluidic-based approaches, a chip comprising microfluidic channels and a CMOS camera may be used. The chip may be characterized by a specific chip geometry, e.g. a square channel cross-section (e.g., in sDC), a rectangular cross-section (e.g., in xDC). Production of exemplary chips (e.g., polydimethylsiloxane, PDMS chip) are described in, e.g., Otto et al. (doi: 10.1038/nmeth.3281) or section 2.2 of Herbig et al. (doi: 10.1007/978-1-4939-7346-0_15) including silicon wafer master preparation and chip production. Further means for detecting are analytical devices which are known to the skilled person.

The term "*microfluidic channel*" as used herein refers to a microfluidic system which transports, mixes, separates, or otherwise processes fluids that are geometrically constrained to a small scale at which surface forces dominate volumetric forces. In a microfluidic system, fluids may be precisely controlled and manipulated by active components (e.g., micropumps or microvalves). The size of the square cross section of a microfluidic channel (e.g., a closed channel) may range between about 100 nm to about 500 $\mu$m.

The term "*fluorescence behaviour*" as used herein refers to signals of fluorescence which may be detected by methods known to the skilled person. Analysis of fluorescence may include determination of background and/or other comparative measurements. In some instances, fluorescence behaviour refers to a cell comprising a heterologous nucleic acid encoding a fluorescent molecule (e.g. a fluorescent chemical compound, a fluorophore or a fluorochrome).

The term "*antibody*" as used herein refers to a ligand that recognizes, binds or interacts with an antigen.

The term "*autoantibody*" as used herein refers to an antibody that recognizes, binds or interacts with an antigen normally found in a subject, a tissue or cell of a subject. Autoantibodies are an example for a biomolecule of interest. For example, an autoimmune disease or disorder is characterized by the production of autoantibodies that attack healthy cells. Autoantibodies may be produced in patients several years before detection of a disease or disorder and can be used as early predictors for risk and possible therapeutic intervention.

The term "*G protein coupled receptor"* or *"GPCR"* as used herein refers to a receptor located at the cell surface which may mediate transmission of intracellular signals by activating guanine nucleotide-binding proteins (G proteins) to which the receptor is coupled. Binding of a specific ligand (e.g., a biomolecule) to a GPCR can cause a conformational change in the receptor, resulting in a form that is able to bind and activate a G protein, thereby triggering a cascade of events that may lead to a biological response.

### *Embodiments*

The details of the present invention are set forth in the description below.

*Detecting biomolecules in a sample by mechanical characterization*

One aspect of the present invention includes the detection of biomolecules in a sample by mechanical characterization wherein the cells being arranged so that the biomolecules to be detected can interact with the cells to thus change one or more mechanical properties of the cell. These properties include cell size, shape, structure, morphology, passage time, mass density, elastic and inelastic light scattering properties, such as Raman and/or Brillouin scattering, dielectric and acoustic impedance, elasticity and/or deformability and/or viscosity. In some instances, the interaction of the biomolecules with the cells changes the fluorescence behaviour of the cells. In some instances, the cells are arranged so that biomolecules of more than one type can interact with the cells (e.g., simultaneously) and different types of biomolecules lead to different detectable changes of properties of the cells.

The step of detecting the properties of the cells may take place in a microfluidic channel, with a liquid test sample being led through the microfluidic channel. Advantages of microfluidic-based approaches are their spectacularly high throughput capabilities. This includes being able to process many cells in a very short time (e.g. at rates of up to 1000 cells per second) as well as detecting several properties of the cells simultaneously (e.g. morphological and rheological parameters). In some instances, several properties of the cells such as physical parameters can be detected or quantified simultaneously.

In some instances, the cell properties are measured by microfluidic-based techniques relying on, e.g., measuring the cell transit time through a microconstriction or on estimating cell deformation caused by hydrodynamic forces. If biomolecules of interest are present in a sample, they interact with a cell by binding to a receptor specific for the biomolecule of interest which results in detectable changes in the mechanical properties of the cell. Preferably, real-time deformability cytometry (RT-DC) is applied to detect the changes in the mechanical properties of the cell at high measurement speed in real time. RT-DC uses a microfluidic chip that constricts the flow of suspended cells. Cells are driven into a funnel-like constriction in a microfluidic channel and are then led into a straight channel where they are deformed by shear forces and pressure gradients into a bullet-like shape, as described in WO 2015/024690 A1 or Figure 1 of Urbanska et al., doi: 10.1016/bs.mcb.2018.06.009. At the end of the channel, the mechanical properties of the cell can be evaluated. In some instances, the microfluidic chip is made of PDMS bonded on cover glass and is connected to at least two inlets (i.e., one for a first syringe with a cell suspension comprising the biomolecules, and one for a second syringe with a measurement buffer) and at least one outlet which is connected by a channel constriction wherein the detection is performed. To quantify mechanical properties of the cell, the microfluidic chip is located on an inverted microscope equipped with a high-speed CMOS camera and a synchronized stroboscopic LED bright-field illumination.

A method of detecting biomolecules in a sample by mechanical characterization (e.g. by real-time deformability cytometry (RT-DC) measurements) comprises the following steps:

  (i) treating mammalian cells with the sample or a control;
  (ii) preparing cell preparation comprising the cells (e.g., at a final concentration of $3\text{-}5 \times 10^6$ cells/mL) in a measurement buffer (MB) containing methylcellulose in a physiological buffer (e.g., at a final concentration of 0.6% (w/v) methylcellulose);
  (iii) preparing a microfluidic chip with a channel width suitable for the cells (e.g., cell diameters may cover 20-90% of the channel width, preferably 30-90% of the channel width);
  (iv) loading the cell preparation into the microfluidic chip;
  (v) performing a measurement of a mechanical property of the cell at a suitable flow rate;
  (vi) performing image processing to determine the mechanical property of the cell (e.g., bright-field images for cell size, deformation, area ratio, and aspect ratio);
  (vii) comparing the detected mechanical property of the sample cell preparation with the reference value of the control cell preparation to infer the presence or absence of the biomolecules.

In step (ii) of the method of detecting biomolecules in a sample by mechanical characterization by RT-DC measurements, the measurement buffer (MB) may be viscosity-adjusted (e.g., at 23-25°C, final viscosity is adjusted to 25 mPa s for blood and bone marrow measurements; as described in Table 3 of Herbig et al., doi: 10.1007/978-1-4939-7346-0_15), osmolarity-adjusted (e.g. standard cell lines or primary cells derived from human or mouse, an MB osmolarity of $320 \pm 20$ mOsm/kg is adequate) and pH controlled (e.g., pH 7.4). Only blood cells and bone marrow cells need to be measured in higher concentration than 0.5% because of fast sedimentation. Otherwise, any concentration between 0.4-1% can be used. For example, 0.6 % (w/v) methylcellulose are dissolved in PBS, thoroughly mixed, and filtered. It is to be noted that other buffers such as serum or cell media can also be used. As RT-DC is made for cells in suspension, adherent cells need to be detached first by using, e.g., trypsin.

In step (v) of the method of detecting biomolecules in a sample by mechanical characterization by RT-DC measurements, the flow rate may be adjusted according to microfluidic chip size (channel width) and viscosity of the measurement buffer (MB). In some instances, channel widths are between 10 to 40 μm and cell

sizes are between 2 to 40 µm (cell diameter) or between 5 to 1050 µm² (cell area). For a channel width of 10 µm, cell sizes between 3 and 9 µm (diameter) or between 7 to 64 µm² (area) are preferred. For a channel width of 10 µm and a viscosity of the MB of 15 mPa s, a total flow rate between 0.004 to 0.016 µL/s, a sample flow rate between 0.001 to 0.004 µL/s and a sheath flow rate between 0.003 to 0.012 µL/s is preferred. For a channel width of 20 µm, cell sizes between 6 and 18 µm (diameter) or between 28 to 254 µm² (area) are preferred. For a channel width of 20 µm and a viscosity of the MB of 15 mPa s, a total flow rate between 0.04 to 0.12 µL/s, a sample flow rate between 0.01 to 0.03 µL/s and a sheath flow rate between 0.03 to 0.12 µL/s is preferred. For a channel width of 30 µm, cell sizes between 9 and 27 µm (diameter) or between 28 to 254 µm² (area) are preferred. For a channel width of 30 µm and a viscosity of the MB of 15 mPa s, a total flow rate between 0.16 to 0.32 µL/s, a sample flow rate between 0.04 to 0.08 µL/s and a sheath flow rate between 0.12 to 0.24 µL/s is preferred., For a channel width of 15 µm and a viscosity of MB of 25mPa.s, a total flow rate between 0.008 to 0.024 µL/s, a sample flow rate between 0.002 to 0.006 µL/s and a sheath flow rate between 0.006 to 0.018µL/s is preferred. For a channel width of 20 µm and a viscosity of MB of 25mPa.s, a total flow rate between 0.02 to 0.06 µL/s, a sample flow rate between 0.005 to 0.015 µL/s and a sheath flow rate between 0.015 to 0.045µL/s is preferred. For a channel width of 30 µm and a viscosity of MB of 25mPa.s, a total flow rate between 0.08 to 0.16 µL/s, a sample flow rate between 0.02 to 0.04 µL/s and a sheath flow rate between 0.06 to 0.12µL/s is preferred. For a channel width of 40 µm and a viscosity of MB of 25mPa.s, a total flow rate between 0.16 to 0.3 µL/s, a sample flow rate between 0.04 to 0.075 µL/s and a sheath flow rate between 0.12 to 0.225µL/s is preferred For a channel width of 40 µm, cell sizes between 12 and 36 µm (diameter) or between 113 to 1018 µm2 (area) are preferred. For a channel width of 40 µm and a viscosity of the MB of 15 mPa s, a total flow rate between 0.32 to 0.96 µL/s, a sample flow rate between 0.08 to 0.24 µL/s and a sheath flow rate between 0.24 to 0.72 µL/s is preferred.

In step (vi) of the method of detecting biomolecules in a sample by mechanical characterization by RT-DC measurements, the image processing may comprise one or more of the following steps, which do not necessarily have to occur in that order: (vi-i) background subtraction; (vi-ii) threshold filtering; (vi-iii) contour finding; (vi-iv) object segmentation, which can be based on pixel classification, and (vi-v) contour processing for the estimation of cell size, position, deformation, and brightness, among others.

For mechanical characterization, cell size, shape, structure, morphology, passage time, mass density, elastic and inelastic light scattering properties, such as Raman and/or Brillouin scattering, dielectric and acoustic impedance, elasticity (elastic modulus, Young's modulus), stiffness, deformability and/or viscosity may be characterized.

The parameter cell size may be measured as diameter in units of µm (cell diameter) and/or as the projected cell cross-sectional area in units of µm² (cell area).

The parameter deformation, d, is based on a feature called circularity, c, and is defined as:

$$d = 1 - c = 1 - \frac{2\sqrt{\pi A}}{P}$$

where A is the area enclosed by the contour (i.e. the projected cross-sectional area of the cell is obtained from the number of pixels within the boundary of the cell, defined by the contour) and P is the perimeter of the contour, e.g., as described in Urbanska et al., doi: 10.1016/bs.mcb.2018.06.009.

The parameter area ratio is defined as:

$$area\ ratio = \frac{A_c}{A_R}$$

where $A_c$ is the area of the convex hull of the detected contour and $A_R$ is the area of the initially detected contour, e.g., as described in Figure 4 of Urbanska et al., doi: 10.1016/bs.mcb.2018.06.009. To determine cell mechanical parameters such as deformation or the derived Young's modulus, the area ratio is preferably set between 1.00 to 1.10, more preferably between 1.00 to 1.05.

The parameter aspect ratio is defined as the ratio between the width and the height of the cell contour's bounding box (x-size/y-size).

In step (vii) of the method of detecting biomolecules in a sample by mechanical characterization by RT-DC measurements, the mechanical properties of the sample cell preparation are compared with the mechanical properties of the control cell preparation (reference value) to infer the presence of the biomolecules. In some instances, statistical modelling may be applied to evaluate a change observed with RT-DC measurement between the sample cell preparation and the control cell preparation (reference value). The exact amount or change in amount is not important if it is statistically significant, as determined by standard techniques.

Changes in deformation of cells exposed to a sample as compared to cells exposed to a control (reference value) may indicate the presence of specific biomolecules in the sample. For example, deformation versus cell size (area) scatter plots derived from an RT-DC measurement may be used to compare the mechanical properties.

In one aspect, the concentration of the detected biomolecule may be evaluated. This can be achieved by correlating the values of one or more quantifiable physical parameters to the specific concentration of the biomolecule to be detected. For example, a method may include a calibration step wherein different biomolecule concentrations are correlated with to one or more physical parameters of the cells.

*Simultaneous detection of two or more different biomolecules*

One aspect of the present invention includes a method of detecting at least two types of biomolecules (e.g. antigenically distinct antibodies) simultaneously in a sample. This includes adding a sample comprising at least two different biomolecules to a cell population comprising at least two cell subpopulations. The first cell subpopulation is arranged so that the first biomolecules to be detected can interact with the cells (feature (i)) and the first cell subpopulation is characterized by a fluorescence behaviour (feature (ii)). The second cell subpopulation is arranged so that the second biomolecules to be detected can interact with the cells (feature (i)) and the second cell subpopulation is characterized by a different fluorescence behaviour as compared to the first cell subpopulation (feature (ii)). Hence, in the cell population, the at least two cell subpopulations differ in features (i) and (ii). Both features may be detected simultaneously, e.g., in a microfluidic channel.

For example, to detect three different biomolecules A, B and C in a sample, a cell population comprising three cell subpopulations A, B and C may be used. Cell subpopulation A expresses (i) receptor A specific for biomolecule A and (ii) a fluorescent molecule A specific for cell subpopulation A. Cell subpopulation B expresses (i) receptor B specific for biomolecule B and (ii) a fluorescent molecule B specific for cell subpopulation B. Cell C expresses (i) receptor C specific for biomolecule C and (ii) a fluorescent molecule C specific for cell subpopulation C. When combining the read-out of the changes in the physical properties of the cells (e.g., mass density, size, or mechanical properties) and the fluorescence behaviour of the cell subpopulations A, B and C, the presence of biomolecules A, B and/or C in the sample may be inferred as compared to a reference value.

A method of detecting at least two different biomolecules in a sample by simultaneous mechanical characterization and flow cytometry-like fluorescence characterization (e.g., by real-time fluorescence and deformability cytometry (RT-FDC) measurements) comprises the following steps:

(i) preparing a cell population (e.g., mammalian cells) comprising at least two cell subpopulations: wherein the first cell subpopulation is arranged so that the first biomolecules to be detected can interact with the cells of the first cell subpopulation, and the first cell subpopulation is characterized by a fluorescence behaviour; and wherein the second cell subpopulation is arranged so that the second biomolecules to be detected can interact with the cells of the second cell subpopulation and the second cell subpopulation is characterized by a different fluorescence behaviour as compared to the first cell subpopulation;
(ii) treating the cell population with the sample or a control wherein the cells in the cell population being arranged so that the biomolecules to be detected can interact with the cells to thus change one or more properties of the cells;
(iii) preparing cell population preparation comprising cells in a measurement buffer (MB) containing methylcellulose in a physiological buffer;
(iv) preparing a microfluidic chip with a channel width suitable for analysing an individual cell of the cell population (e.g., cell diameters may cover 20-90% of the channel width);
(v) loading the cell population preparation into the microfluidic chip;
(vi) performing a simultaneous measurement of cell morphology, cell mechanics (e.g. by bright-field imaging) and cell fluorescence (e.g. by multicolour imaging) for the individual cell of the cell population at a suitable flow rate;
(vii) assigning the individual cell to the first or the second cell subpopulation based on its fluorescence behaviour;
(viii) comparing the detected mechanical property of the first cell population with the reference value to infer the presence or absence of the first biomolecules to be detected; and
(ix) comparing the detected mechanical property of the second cell preparation with the reference value to infer the presence or absence of the second biomolecules to be detected.

In step (vi) of the method of detecting at least two biomolecules in a sample by simultaneous mechanical characterization and flow cytometry-like fluorescence characterization, measuring fluorescence allows to distinguish two or more cell subpopulation by its fluorescence behaviour which is unique for the cell subpopulation (and the receptor for a specific biomolecule expressed in said subpopulation). The cell surface may be labelled with a fluorescent molecule, or the cell may express a heterologous fluorescent molecule (e.g. a fluorescent chemical compound, a fluorophore or fluorochrome). When subpopulations each of which expressing a fluorescent molecule with different spectral property are mixed in a cell population, individual cells can be assigned to their subpopulation by multicolour imaging (e.g., by using a multi-channel scan). To allow spectral discrimination between the cell subpopulations, suitable fluorophores and emission filters may be selected to minimize spectral overlap between the two fluorophore emission profiles to discriminate the spectra. Preferably, emission profiles of the fluorophores are at least 30 nm apart. To distinguish two cell subpopulation, two suitable dyes may be used for two-channel fluorescence experiments.

The present methods may be further combined with standard approaches (e.g., proteomics, transcriptomics, genomics and metagenomics). For example, for clinical diagnosis, methods such as enzyme-linked immunosorbent assay (ELISA), quantitative real-time PCR (RT-qPCR), next generation sequencing (NGS) analysis, rapid tests, point of care (POC) testing, liquid chromatography, and/or mass spectrometry may be used.

*Samples, biomolecules and cells for the present methods*

One aspect of the present invention includes the detection of one or more biomolecules in a sample comprising adding cells to the sample.

A sample may be a fluid, preferably a liquid. A sample may be obtained from a subject such as saliva, blood, serum, excreta (e.g., urine, faeces), semen, perspiration, tears, a body fluid (e.g., cerebrospinal fluid, CSF) and body tissues of a subject. In some instances, a sample may be processed prior to the step of combining the sample with cells (or a cell population) in the present methods, e.g., by filtration, purification, and/or dilution.

The cells used in the present methods may be mammalian cells, more preferably human, mouse, rat, or hamster cells. For example, cells may be myelogenous leukemia cell line (K562, HL60), breast carcinoma cell line (MDA-MB-231), or other cancer cell lines (e.g., H1975, HCC827, TMet, TnonMet, and TMet-Nkx2-1, L1210) etc. The sample and the cell may be of natural origin (e.g., a biological species) or synthetic origin (e.g., a sample not of natural origin). In some instances, a sample and a cell originate from the same biological species (e.g., both are derived from a human), or from different biological species (e.g., a sample derived from a human and a mouse cell).

When cells are added to the sample, the cells are arranged so that the biomolecules to be detected can interact with the cells to thus change one or more properties of the cells. In some instances, an interaction of a biomolecule in a sample and a cell is mediated by a receptor. The receptor may be specific for a type of biomolecule (e.g., a protein), a class of biomolecules (e.g., antibodies), or an individual biomolecule (e.g., an autoantibody with a specific glycosylation pattern). A mammalian cell may express a natural receptor such as a cell surface molecule including its natural conformation and posttranslational modifications (e.g., phosphorylation, ubiquitination, glycosylation, palmitoylation). For example, a natural receptor may be a signalling receptor such as G-protein coupled receptor (GPCR) that regulates physiological responses of a cell. A synthetic receptor may be derived from a natural receptor, e.g., by engineering residues for increased affinity or specificity for its target biomolecule. A cell may be modified to express the synthetic receptor, e.g., by genome editing.

In some instances, the biomolecules are antibodies interacting with a receptor of the cells, preferably a cell surface receptor. In some instances, the antibodies are autoantibodies. In some cases, the method for detecting a specific autoantibody is performed by using mammalian cells expressing a natural or recombinant receptor for the specific autoantibody. In some instances, to detect autoantibodies, cells expressing G-protein coupled receptors (GPCRs) such as β2-adrenoceptor (β2-fAAB), the α1-adrenoceptor (α1-fAAB), the angiotensin II AT1-receptor (AT1-fAAB), and the nociception-like opioid receptor (NOC-fAAB), muscarinic M2-receptor (M2-fAAB), the MAS-receptor (MAS-fAAB), and the ETA-receptor (ETA-fAAB) may be used.

One aspect of the present invention includes a cell that is arranged for changing at least one of its properties when exposed to biomolecules, wherein the at least one of its properties of the cell can be measured by physical phenotyping, and wherein the cell is arranged for maintaining a different property which is specific for the modified cell when exposed to the biomolecules. In some instances, the physical phenotyping is performed in less than 0.001 seconds. In some instances, the property which is maintained (i.e. unchanged or not statistically significantly changed between sample and control) is fluorescence behaviour of the cell. In some instances, the cell has a smooth contour and a cell size (measured as the projected cell cross-sectional area in units of $\mu m^2$) between 25 to 225 $\mu m^2$, preferably between 100 to 175 $\mu m^2$. However, it is not necessary that the contour is smooth, and one may even not have to detect a contour in order to calculate the cell size.

*Medical applications of the present methods*

One aspect of the present invention includes detecting biomolecules in a sample for diagnostic applications. The samples may be collected from the same or different body fluids of a subject over a time series (e.g., several times per day, each day for several weeks, or at defined intervals for longer periods such as months or years) to monitor changes in the presence or concentration of a specific biomolecule and/or changes in biomolecule composition (e.g., an increase of biomolecule A accompanied by a decrease of biomolecule B).

In some instances, biomolecules are detected in a sample for monitoring disease progression or response to a treatment in a subject. In some instances, the samples are collected before and after a treatment of a subject, e.g., a medical treatment. In cases wherein a subject has been previously determined to have a disease or disorder, the present methods may be used to identify a therapy suitable for the disease or disorder, for example by detecting changes in at least one property of the cells after administering a medical treatment in an amount and for a duration sufficient to treat the disease or disorder. For example, a method comprising steps of obtaining a sample from a subject's body fluid (e.g., saliva, blood, urine or cerebrospinal fluid), preparing the obtained sample for analysis, combining the sample with cells, detecting at least one property of the cells, and inferring the presence, absence or concentration of biomolecules in the sample.

In some instances, subjects tested negative for a disease or subjects prior to a disease may be used as a control sample to infer a reference value for the step of comparing the detected properties of the cells with reference values to infer to presence or absence of a biomolecule. In some instances, if a sex difference in the response to a disease is known, the control may be adjusted accordingly. In some instances, subjects tested negative for COVID-19 disease (e.g., by RT-qPCR of nasopharyngeal swab samples) may be used as a control for detecting biomolecules such as autoantibodies in a sample of a patient suffering from Long-COVID-19 (PASC).

Compared to healthy control subjects, subjects suffering from an autoimmune disorder show enriched autoantibodies levels, including autoimmune disorders such as systemic lupus erythematosus (e.g., anti-interferon-a (IFN-$\alpha$) autoantibodies), idiopathic inflammatory myopathies such as sporadic inclusion body myositis (e.g., anti-cytosolic 5'-nucleosidase 1A (anti-cN1A) autoantibodies), encephalitis, rheumatoid arthritis (e.g., anti-ADAM33, anti-Honerin, anti-HCN3 autoantibodies), multiple sclerosis (e.g., anti-Kir4.1 autoantibodies), interstitial lung disease (e.g., anti-BPIFB1 autoantibodies), type I diabetes (e.g., anti-Znt8, anti-GAD65, anti-IA2 autoantibodies), scleroderma (e.g., anti-RPC1 autoantibodies), systemic autoimmune diseases (e.g., anti-Ro52, anti-Ro60, anti-RNP-70 autoantibodies), disseminated non-tuberculosis mycobacterial infection (e.g., anti-interferon-$\gamma$ (IFN-y) autoantibodies), or autoimmune thyroid disease such as Graves' disease or Hashimoto's thyroiditis (e.g., anti-TSH-receptor (TRAb) autoantibodies). Other factors (e.g., environmental factors or diseases) may also impact the level of autoantibodies in a subject.

In one aspect, the system and method may be used for detecting autoantibodies in a body fluid obtained from a subject (e.g., an animal disease model or a patient) which is suspected of having a disease or disorder, or which was exposed to a specific treatment (e.g., to study progress of a therapeutic treatment such response to a drug or after immunisation by vaccination). The system for detecting the autoantibodies may comprise a microfluidic channel, cells expressing a receptor for the autoantibodies and a detector of the cell's properties which change upon interaction of the receptor and the autoantibodies. After sample preparation, the cells are combined with the sample before or during their passage in the microfluidic channel. Subsequently, the properties of the cells are measured and compared to properties of a control measurement and/or control values (reference value). If one or more properties of the cells are changed in comparison to the reference value, the presence of autoantibodies is detected.

For diagnostic applications and methods of therapy and treatment, biomolecules may be a marker a disease or disorder. For example, patients suffering from Long-COVID-19 have increased levels of autoantibodies (e.g., anti-GPCR autoantibodies; see [7] Wallukat et al., doi: 10.1016/j.jtauto.2021.100100). Detecting such anti-GPCR autoantibodies is also encompassed by the present methods.

A kit or a cell may be used in the method of detecting a biomolecule in a sample obtained from a patient, preferably wherein the sample is saliva, blood, urine, or cerebrospinal fluid (CSF).

## Experimental section

The following are examples of systems and methods disclosed herein. It is understood that various other aspects may be practiced, given the general and detailed descriptions provided elsewhere herein.

### Biomolecule detection by measuring changes in cell deformability (Figure 4)

For biomolecule detection in a sample, the **presence or absence of a biomolecule of interest** (e.g., autoantibodies) may be detected by mechanical phenotyping of mammalian cells with a sample comprising the biomolecule of interest (e.g., autoantibodies). High-throughput mechanical phenotyping may be performed by real-time deformability cytometry (RT-DC) which allows for on-the-fly analysis of deformed cell shape.

*Samples.* Sample 1, vehicle for reference value: water. Sample 2, beta2 agonist. Sample 3, human serum comprising autoantibodies targeting G-protein coupled receptors (GPCRs) such as the beta2-adrenoceptor: blood samples were collected from Long-COVID-19 patients, after recovery from acute disease. Patient sera were isolated.

Cells. The experiments were done on HL60 cells, a myeloid precursor cell line, which has served as a standard suspended cell line in comparable experiments with an optical stretcher. Where appropriate, results were confirmed with primary cells. The HL60 cell line was originally derived from a patient suffering from APL in 1977 and modified to the current HL60/S4 line (a generous gift from D. and A. Olins of the Department of Pharmaceutical Sciences, College of Pharmacy, University of New England, who had confirmed the cell line before sending) used in this work. Cells were cultured in RPMI-1640 medium (Life Technologies) with 10% FCS (Life Technologies) and 1% penicillin/streptomycin (Life Technologies) in a standard incubator (Hera Cell, Thermo Scientific) at 37 °C, 5% $CO_2$ and 95% air. The cells were split every 48 h and resuspended to a concentration of $2 \times 10^5$ cells/ml. For experiments, cells were taken during log phase, approximately 36 h after splitting. Cells were initially checked for mycoplasma contamination (Mycoplasma Kit, Sigma-Aldrich) and regularly renewed from frozen stocks.

*Cell culture.* After thawing, cells were equilibrated for several days in cell culture using appropriate cell culture medium and cell culture conditions. Cells were split between a T25 flask and a 6-well plate for RT-DC measurement and the treatment. Each condition was measured in triplicates (e.g. three wells of a 6-well plate). About 24 hours prior to RT-DC measurement, cell numbers were adjusted to $1{,}0 \times 10^5$ cells/mL with a total volume of 2,5 mL/well.

*Combining cells with samples (treatment).* Human HL60 cells were incubated with reference (sample 1) or a beta2-adrenoceptor agonist (sample 2 or 3). Cell numbers were adjusted to a final concentration of $5 \times 10^6$ cells/mL. Cells were transferred from each well into an appropriate tube for treatment. For the sample 1 (reference): the same amount of vehicle (e.g. water) as used for the drug (e.g., β2 agonist). For sample 2 (drug, beta2 agonist): an appropriate volume of working concentration was added to the cell suspension. For sample 3 (human serum containing anti-beta2 receptor): the sample was added to a final volume of about 10% v/v, e.g., 10 μL of sample to 100 μL cell suspension. For all samples 1 to 3, treatment was performed for 10 min at room temperature. After treatment, cells were collected by centrifugation (e.g. 200 × g for 3 min).

Next, an **RT-DC measurement** was performed to detect changes in cell deformability in samples 2 and 3 as compared to sample 1 (reference).

For the measurement, 0.6 % MC-PBS measurement buffer (MB) was used prepared and filtered through a 0.22 μm filter prior to use. Microfluidic chips with measurement channel dimensions of 30 × 30 μm (30 μm channel width) were used. For each sample / condition a new chip was used. For each sample / condition, the cell pellet was resuspended in 0.6 % MC-PBS measurement buffer (MB), e.g., 50 μL of 0.6% MC-PBS by avoiding the introduction of bubbles.

Suitable measurement conditions were selected for the RT-DC device. Measurement of cell-MB suspension was performed at a total flow rate of 0.1200 μL/s (equilibrate flow for at least 2 min). As internal control, a reservoir measurement (as described in, e.g., Figure 4C of Herbig et al., doi: 10.1007/978-1-4939-7346-0_15) was performed, followed by a channel measurement at the end of the measurement channel. Bright-field images were captured by a high-speed camera which was mounted to one port of the microscope and has a CMOS sensor. The camera triggered the LED light source (suitable LED shutter time selected to minimize motion blurring).

*Detection of changes.* Deformability cytometry plots of HL60 cells (deformation versus cell size (area) scatter plots derived from an RT-DC measurement) show the deformation for each cell event with its corresponding measured cell area ($\mu m^2$).

*Results.* Deformability cytometry plots indicated a smaller cell deformation for cells treated with a GPCR agonist (a drug activating GPCR in sample 2, or patient serum comprising GPCR autoantibodies in sample 3) as compared to control cells, as shown in contour plots of **Figure 4(d).**

**Industrial applicability**

The present system and methods are reliable and deal effectively with biomolecule detection for monitoring, biochemical research, medical diagnosis, predicting therapeutic responses, understanding disease pathogenesis, and drug development. Thus, the present system and methods are industrially applicable.

Further applications and practical exploitation in industry may be derived from the present description by the skilled person's general knowledge.

**List of references**

[1] E. Drucker and K. Krapfenbauer, "Pitfalls and limitations in translation from biomarker discovery to clinical utility in predictive and personalised medicine," 2013, doi: 10.1186/1878-5085-4-7.

[2] R. Mayeux, "Biomarkers: Potential Uses and Limitations," NeuroRx, vol. 1, no. 2, pp. 182-188, 2004, doi: 10.1602/neurorx.1.2.182.

[3] S. B. Nimse, M. D. Sonawane, K.-S. Song, and T. Kim, "Analyst CRITICAL REVIEW Biomarker detection technologies and future directions," Cite this Anal., vol. 141, p. 740, 2016, doi: 10.1039/c5an01790d.

[4] P.-H. Wu et al., "A comparison of methods to assess cell mechanical properties," Nat. Methods, 2018, doi: 10.1038/s41592-018-0015-1.

[5] M. Urbanska et al., "A comparison of microfluidic methods for high-throughput cell deformability measurements," Nat. Methods, vol. 17, no. 6, pp. 587-593, 2020, doi: 10.1038/s41592-020-0818-8.

[6] Burbelo and O'Hanlon, "New Autoantibody Detection Technologies Yield Novel Insights into Autoimmune Disease", Curr Opin Rheumatol. 2014, doi: 10.1097/BOR.0000000000000107

[7] Wallukat et al., "Functional autoantibodies against G-protein coupled receptors in patients with persistent Long-COVID-19 symptoms", Journal of Translational Autoimmunity 4, 2021, doi: 10.1016/j.jtauto.2021.100100

[8] Morgenthaler et al., "Application of a bioassay with CHO cells for the routine detection of stimulating and blocking autoantibodies to the TSH-receptor", Horm Metab Res, 1998, doi: 10.1055/s-2007-978858

[9] Otto et al., "Real-time deformability cytometry:

on-the-fly cell mechanical phenotyping". Nat. Methods, 2015, doi: 10.1038/nmeth.3281)

[10] Herbig et al., "Real-Time Deformability Cytometry: Label-Free Functional Characterization of Cells", Humana Press, 2018 (doi: 10.1007/978-1-4939-7346-0_15)

[11] Al-Aly et al., Nature 2021, "High-dimensional characterization of post-acute sequelae of COVID-19", doi: 10.1038/s41586-021-03553-9

[12] WO 2015/024690 A1, "Apparatus and method for determining the mechanical properties of cells"

[13] Urbanska et al., chapter 10, "High-throughput single-cell mechanical phenotyping with real-time deformability cytometry", Methods in Cell Biology, pages 175-198, Volume 147, ISSN 0091-679X, 2018 Elsevier Inc. (doi: 10.1016/bs.mcb.2018.06.009)

[14] Wallukat et al., Anti-β1-Adrenoceptor Autoantibodies with Chronotropic Activity from the Serum of Patients with Dilated Cardiomyopathy: Mapping of Epitopes in the First and Second Extracellular Loops, J. Mol. Cell Cardiol. 27, 397-406.

**Claims**

1. A method of detecting biomolecules in a sample, the method comprising:

   - adding cells to the sample, the cells being arranged so that the biomolecules to be detected can interact with the cells to thus change one or more properties of the cells,
   - detecting the properties of the cells, and
   - comparing the detected properties with reference values to infer the presence or absence and/or concentration of the biomolecules.

2. The method according to claim 1, wherein the detection of the properties of the cells takes place in a microfluidic channel, with the sample being led through the microfluidic channel, wherein preferably, the detected properties comprise mechanical properties, and wherein the cells are preferably deformed inside the channel.

3. The method according to claim 1 or 2, wherein the cells are arranged so that the interaction of the biomolecules with the cells is binding of the biomolecules to a receptor of the cells.

4. The method according to one of the preceding claims, wherein the cells are arranged so that more than one type of biomolecules can interact with the cells and wherein different types of biomolecules lead to different detectable changes of properties of the cells.

5. The method according to one of the preceding claims, wherein the properties include one or more of the size, shape, structure, morphology, passage time, mass density, elastic and inelastic light scattering properties, such as Raman and/or Brillouin scattering, dielectric and acoustic impedance and/or deformability, elasticity and viscosity of the cells.

6. The method according to one of the preceding claims, wherein the cells are mammalian cells, and optionally wherein the cells comprise or consist of natural cells, transfected cells, and/or synthetic cells.

7. The method according to one of the preceding claims, wherein the cells are configured to change their properties when exposed to the biomolecules, and wherein the biomolecules are antibodies interacting with a receptor of the cells, preferably a cell surface molecule, optionally wherein the antibodies are autoantibodies that target G protein coupled receptors (GPCRs).

8. The method according to one of the preceding claims, further comprising using the detected properties to infer the concentration of biomolecules in the sample.

9. The method according to one of the preceding claims, wherein the sample is a fluid, preferably a liquid;

   optionally wherein the fluid is obtained from a subject, further optionally wherein the fluid is saliva, blood, excreta, and/or a body fluid, preferably wherein the excreta is urine, and/or wherein the body fluid is cerebrospinal fluid (CSF); and optionally wherein the subject is a mammal.

10. A method of detecting at least two types of biomolecules in a sample, the method comprising:

    - adding a cell population to the sample; wherein the cell population comprises at least two cell subpopulations or at least two groups of cells from different originas:

      the first cell subpopulation/group is arranged so that the first type of biomolecules to be detected can interact with cells of the first cell subpopulation/group, and the first cell subpopulation/group is **characterized by** a fluorescence behaviour, and the second cell subpopulation/group is arranged so that the second type of biomolecules to be detected can interact with cells of the second cell subpopulation/group and the second cell subpopulation/group is **characterized by** a different fluorescence behaviour as compared to the first cell subpopulation/group; and
wherein the cells being arranged so that the biomolecules to be detected can interact with the cells to thus change one or more properties of the cells;

- detecting the properties of the cells and the fluorescence behaviour of the cells;
- comparing the detected properties with reference values to infer the presence or absence of the biomolecules;
- comparing the fluorescence behaviour of the cells to assign the change of the detected properties to the first or the second type of cells; and
- to infer the presence or absence of the first type of biomolecules and the presence or absence of the second type of biomolecules.

11. The method according to claim 10, wherein the types of biomolecules are biomolecules which interact differently with the cells,
optionally wherein the biomolecules bind different receptors of the cell.

12. The method according to claim 10 or 11, wherein the biomolecules are antibodies and the types of biomolecules are antigenically distinct antibodies, wherein the first type of antibody can interact with cells of the first cell subpopulation/group but not with cells of the second cell subpopulation/group, and the second type of antibody can interact with cells of the second cell subpopulation/group but not with cells the first cell subpopulation/group.

13. The method according to one of claims 10 to 12, wherein the detected properties of the cells are mechanical properties, and wherein the mechanical properties and the fluorescence behaviour of the cells are detected in a microfluidic channel.

14. A kit arranged for detecting biomolecules, comprising:

- one or more cells, the cells being arranged so that the biomolecules to be detected can interact with the cells to thus change one or more properties of the cells,
- a means for detecting the one or more properties of the cells, the kit preferably being arranged for being used in the method of one of the preceding claims.

15. The kit for use in a method of detecting one or more biomolecules in a sample obtained from a subject, preferably wherein the sample is saliva, blood, urine, or cerebrospinal fluid (CSF).

**Amended claims in accordance with Rule 137(2) EPC.**

1. A method of detecting biomolecules in a sample, the method comprising:

- adding cells to the sample, the cells being arranged so that the biomolecules to be detected can interact with the cells to thus change one or more properties of the cells,
- detecting the properties of the cells, and
- comparing the detected properties with reference values to infer the presence or absence and/or concentration of the biomolecules;

wherein the detection of the properties of the cells takes place in a microfluidic channel, with the sample being led through the microfluidic channel.

2. The method according to claim 1, wherein the detected properties comprise mechanical properties, and wherein the cells are preferably deformed inside the channel.

3. The method according to claim 1 or 2, wherein the cells are arranged so that the interaction of the biomolecules with the cells is binding of the biomolecules to a receptor of the cells.

4. The method according to one of the preceding claims, wherein the cells are arranged so that more than one type of biomolecules can interact with the cells and wherein different types of biomolecules lead to different detectable changes of properties of the cells.

5. The method according to one of the preceding claims, wherein the properties include one or more of the size, shape, structure, morphology, passage time, mass density, elastic and inelastic light scattering properties, such as Raman and/or Brillouin scattering, dielectric and acoustic impedance and/or deformability, elasticity and viscosity of the cells.

6. The method according to one of the preceding claims, wherein the cells are mammalian cells, and optionally wherein the cells comprise or consist of natural cells, transfected cells, and/or synthetic cells.

7. The method according to one of the preceding claims, wherein the cells are configured to change their properties when exposed to the biomolecules, and wherein the biomolecules are antibodies interacting with a receptor of the cells, preferably a cell surface molecule, optionally wherein the antibodies are autoantibodies that target G protein coupled receptors (GPCRs).

8. The method according to one of the preceding claims, further comprising using the detected properties to infer the concentration of biomolecules in the sample.

9. The method according to one of the preceding claims, wherein the sample is a fluid, preferably a liquid;

   optionally wherein the fluid is obtained from a subject, further optionally wherein the fluid is saliva, blood, excreta, and/or a body fluid, preferably wherein the excreta is urine, and/or wherein the body fluid is cerebrospinal fluid (CSF); and optionally wherein the subject is a mammal.

10. A method of detecting at least two types of biomolecules in a sample, the method comprising:

    - adding a cell population to the sample; wherein the cell population comprises at least two cell subpopulations or at least two groups of cells from different origins:

      the first cell subpopulation/group is arranged so that the first type of biomolecules to be detected can interact with cells of the first cell subpopulation/group, and the first cell subpopulation/group is **characterized by** a fluorescence behaviour, and the second cell subpopulation/group is arranged so that the second type of biomolecules to be detected can interact with cells of the second cell subpopulation/group and the second cell subpopulation/group is **characterized by** a different fluorescence behaviour as compared to the first cell subpopulation/group; and wherein the cells being arranged so that the biomolecules to be detected can interact with the cells to thus change one or more properties of the cells;

    - detecting the properties of the cells and the fluorescence behaviour of the cells;
    - comparing the detected properties with reference values to infer the presence or absence of the biomolecules;
    - comparing the fluorescence behaviour of the cells to assign the change of the detected properties to the first or the second type of cells; and
    - to infer the presence or absence of the first type of biomolecules and the presence or absence of the second type of biomolecules; wherein the detected properties of the cells are mechanical properties, and wherein the mechanical properties and the fluorescence behaviour of the cells are detected in a microfluidic channel.

11. The method according to claim 10, wherein the types of biomolecules are biomolecules which interact differently with the cells, optionally wherein the biomolecules bind different receptors of the cell.

12. The method according to claim 10 or 11, wherein the biomolecules are antibodies and the types of biomolecules are antigenically distinct antibodies, wherein the first type of antibody can interact with cells of the first cell subpopulation/group but not with cells of the second cell subpopulation/group, and the second type of antibody can interact with cells of the second cell subpopulation/group but not with cells of the first cell subpopulation/group.

13. A kit arranged for detecting biomolecules, comprising:

    - one or more cells, the cells being arranged so that the biomolecules to be detected can interact with the cells to thus change one or more properties of the cells,
    - a means for detecting the one or more properties of the cells, the kit being arranged for being used in a method comprising:
    - adding the cells to the sample,
    - detecting the properties of the cells, and
    - comparing the detected properties with reference values to infer the presence or absence and/or concentration of the biomolecules;

    wherein the detection of the properties of the cells takes place in a microfluidic channel, with the sample being led through the microfluidic channel.

14. The kit according to claim 13, wherein the kit is arranged for being used in the method according to any one of claims 2 to 12.

15. The kit according to claim 13 or 14 for use in a method of therapy or a diagnostic method, wherein one or more biomolecules are detected in a sample obtained from a subject, preferably wherein the sample is saliva, blood, urine, or cerebrospinal fluid (CSF).

Figure 1/4

Figure 2/4

Figure 3/4

Figure 4/4

EP 4 242 658 A1

Europäisches
Patentamt

European
Patent Office

Office européen
des brevets

**EUROPEAN SEARCH REPORT**

Application Number

EP 22 16 1335

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Davideit Hanna ET AL: "Determination of Agonistically Acting Autoantibodies to the Adrenergic Beta-1 Receptor by Cellular Bioassay : Methods and Protocols" In: "Peptide Synthesis— Methods and Protocols", 1 January 2019 (2019-01-01), Springer New York, New York, NY, XP055955008, ISSN: 1064-3745 ISBN: 978-1-0716-0229-4 vol. 1901, pages 95-102, DOI: 10.1007/978-1-4939-8949-2_8, Retrieved from the Internet: URL:http://link.springer.com/content/pdf/10.1007/978-1-4939-8949-2_8> * abstract * | 1-15 | INV. G01N33/543 G01N33/50 |
| X,D | WALLUKAT GERD ET AL: "Functional autoantibodies against G-protein coupled receptors in patients with persistent Long-COVID-19 symptoms", JOURNAL OF TRANSLATIONAL AUTOIMMUNITY, vol. 4, 1 January 2021 (2021-01-01), page 100100, XP055954877, ISSN: 2589-9090, DOI: 10.1016/j.jtauto.2021.100100 * page 2 – page 4; figure 1 * | 1-15 | TECHNICAL FIELDS SEARCHED (IPC) G01N |

-/--

The present search report has been drawn up for all claims

| Place of search | Date of completion of the search | Examiner |
|---|---|---|
| Munich | 29 August 2022 | Gonçalves Mauger, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Application Number

EP 22 16 1335

**EUROPEAN SEARCH REPORT**

| | DOCUMENTS CONSIDERED TO BE RELEVANT | | |
|---|---|---|---|
| Category | Citation of document with indication, where appropriate, of relevant passages | Relevant to claim | CLASSIFICATION OF THE APPLICATION (IPC) |
| X | OTTO O ET AL: "Real-time deformability cytometry as a label-free indicator of cell function", 2015 37TH ANNUAL INTERNATIONAL CONFERENCE OF THE IEEE ENGINEERING IN MEDICINE AND BIOLOGY SOCIETY (EMBC), IEEE, 25 August 2015 (2015-08-25), pages 1861-1864, XP032810523, DOI: 10.1109/EMBC.2015.7318744 [retrieved on 2015-11-04] * see abstract; page 1862 - page 1864; figures * ----- | 1-15 | |
| X | WENZEL KATRIN ET AL: "Performance and in-house validation of a bioassay for the determination of beta1-autoantibodies found in patients with cardiomyopathy", HELIYON, vol. 3, no. 7, 1 July 2017 (2017-07-01), page e00362, XP055955012, GB ISSN: 2405-8440, DOI: 10.1016/j.heliyon.2017.e00362 * page 4 - page 9 * ----- -/-- | 1-15 | TECHNICAL FIELDS SEARCHED (IPC) |

The present search report has been drawn up for all claims

| Place of search | Date of completion of the search | Examiner |
|---|---|---|
| Munich | 29 August 2022 | Gonçalves Mauger, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

Europäisches
Patentamt

European
Patent Office

Office européen
des brevets

**EUROPEAN SEARCH REPORT**

Application Number

EP 22 16 1335

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WALLUKAT GERD ET AL: "Autoantibodies directed against [alpha]1-adrenergic receptor and endothelin receptor A in patients with prostate cancer", AUTOIMMUNITY HIGHLIGHTS, vol. 11, no. 1, 20 September 2020 (2020-09-20), XP055955017, Cham ISSN: 2038-0305, DOI: 10.1186/s13317-020-00136-y Retrieved from the Internet: URL:https://link.springer.com/content/pdf/10.1186/s13317-020-00136-y.pdf> * page 2 - page 4; figure 1 * | 1-15 | |
| X | JOSHI-BARR SHIVANJALI ET AL: "High throughput bioassay for beta1-adrenoceptor autoantibody detection", INTERNATIONAL JOURNAL OF CARDIOLOGY, ELSEVIER, AMSTERDAM, NL, vol. 219, 8 June 2016 (2016-06-08), pages 98-104, XP029655634, ISSN: 0167-5273, DOI: 10.1016/J.IJCARD.2016.06.002 * page 99 - page 101 * | 1-15 | TECHNICAL FIELDS SEARCHED (IPC) |

-/--

The present search report has been drawn up for all claims

| Place of search | Date of completion of the search | Examiner |
|---|---|---|
| Munich | 29 August 2022 | Gonçalves Mauger, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

**EP 22 16 1335**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Urbanska Marta ET AL: "High-throughput single-cell mechanical phenotyping with real-time deformability cytometry" In: "Cytometry: Part B", 1 January 2018 (2018-01-01), San Diego [u.a.] : Acad. Press, US, XP055955034, ISSN: 0091-679X vol. 147, pages 175-198, DOI: 10.1016/bs.mcb.2018.06.009, Retrieved from the Internet: URL:http://dx.doi.org/10.1016/bs.mcb.2018.06.009> | 1,14,15 | |
| A | * pages 178, 193 – page 195; figure 1 * | 2-13 | |

TECHNICAL FIELDS
SEARCHED      (IPC)

The present search report has been drawn up for all claims

| Place of search | Date of completion of the search | Examiner |
|---|---|---|
| Munich | 29 August 2022 | Gonçalves Mauger, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 4 of 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015024690 A1

### Non-patent literature cited in the description

- **WALLUKAT et al.** Anti-β1-Adrenoceptor Autoantibodies with Chronotropic Activity from the Serum of Patients with Dilated Cardiomyopathy: Mapping of Epitopes in the First and Second Extracellular Loops. *J. Mol. Cell Cardiol.,* vol. 27, 397-406
- **E. DRUCKER ; K. KRAPFENBAUER.** *Pitfalls and limitations in translation from biomarker discovery to clinical utility in predictive and personalised medicine,* 2013
- **R. MAYEUX.** Biomarkers: Potential Uses and Limitations. *NeuroRx,* 2004, vol. 1 (2), 182-188
- **S. B. NIMSE ; M. D. SONAWANE ; K.-S. SONG ; T. KIM.** Analyst CRITICAL REVIEW Biomarker detection technologies and future directions. *Cite this Anal.,* 2016, vol. 141, 740
- **P.-H. WU et al.** A comparison of methods to assess cell mechanical properties. *Nat. Methods,* 2018
- **M. URBANSKA et al.** A comparison of microfluidic methods for high-throughput cell deformability measurements. *Nat. Methods,* 2020, vol. 17 (6), 587-593
- **BURBELO ; O'HANLON.** New Autoantibody Detection Technologies Yield Novel Insights into Autoimmune Disease. *Curr Opin Rheumatol,* 2014
- **WALLUKAT et al.** Functional autoantibodies against G-protein coupled receptors in patients with persistent Long-COVID-19 symptoms. *Journal of Translational Autoimmunity,* 2021, vol. 4
- **MORGENTHALER et al.** Application of a bioassay with CHO cells for the routine detection of stimulating and blocking autoantibodies to the TSH-receptor. *Horm Metab Res,* 1998
- **OTTO et al.** Real-time deformability cytometry: on-the-fly cell mechanical phenotyping. *Nat. Methods,* 2015
- **HERBIG et al.** Real-Time Deformability Cytometry: Label-Free Functional Characterization of Cells. Humana Press, 2018
- **AL-ALY et al.** High-dimensional characterization of post-acute sequelae of COVID-19. *Nature,* 2021
- High-throughput single-cell mechanical phenotyping with real-time deformability cytometry. **URBANSKA et al.** Methods in Cell Biology. Elsevier Inc, vol. 147, 175-198
- **WALLUKAT et al.** Anti-β1-Adrenoceptor Autoantibodies with Chronotropic Activity from the Serum of Patients with Dilated Cardiomyopathy: Mapping of Epitopes in the First and Second Extracellular Loops. *J. Mol. Cell Cardiol,* vol. 27, 397-406